Europäisches Patentamt

European Patent Office

Office européen des brevets

(11) Publication number: 0 380 334
A2

# EUROPEAN PATENT APPLICATION

(21) Application number: 90300772.2

(22) Date of filing: 25.01.90

(51) Int. Cl.⁵: A61K 37/465, A61K 9/127

(30) Priority: 27.01.89 JP 16406/89
17.05.89 JP 121405/89

(43) Date of publication of application:
01.08.90 Bulletin 90/31

(84) Designated Contracting States:
BE CH DE DK ES FR GB IT LI NL SE

(71) Applicant: THE GREEN CROSS CORPORATION
3-3, Imabashi 1-chome Chuo-ku
Osaka-shi Osaka(JP)

(72) Inventor: Matsuda, Hiroshi, c/o Chuo
Kenkyusho
The Green Cross Corporation, 1180-1
Shodaiotani-2-chome, Kirakata-shi(JP)
Inventor: Ueda, Yasuo, c/o Chuo Kenkyusho
The Green Cross Corporation, 1180-1
Shodaiotani-2-chome, Kirakata-shi(JP)
Inventor: Tamanouchi, Kouichi, c/o Chuo
Kenkyusho
The Green Cross Corporation, 1180-1
Shodaiotani-2-chome, Kirakata-shi(JP)

(74) Representative: Coleiro, Raymond et al
MEWBURN ELLIS 2 Cursitor Street
London EC4A 1BQ(GB)

(54) Urokinase precursor-lipid composite.

(57) A urokinase precursor-lipid composite comprises urokinase precursor and a lipid. Such composites are useful as thrombolytic agent.

EP 0 380 334 A2

## UROKINASE PRECURSOR-LIPID COMPOSITE

### BACKGROUND OF THE INVENTION

#### FIELD OF THE INVENTION

This invention relates to a urokinase precursor-lipid composite comprising urokinase precursor and a lipid.

#### DESCRIPTION OF THE PRIOR ART

The urokinase precursor secreted from the cells of human kidney exhibits no fibrinolytic activity in itself. However, when it undergoes the action of a proteinase such as plasmin, it is transformed into urokinase and exhibits a marked fibrinolytic activity.

Having a high affinity to fibrin, urokinase precursor arrives at the fibrin constituting a thrombus without acting (decomposing) upon the fibrinogen in plasma, and exhibits a fibrinolytic activity under the action of slight quantity of plasmin present in thrombus.

Thus, urokinase precursor causes a fibrinolysis only in the site of thrombus and selectively and effectively dissolves thrombus. Accordingly, it is considered hopeful as a novel agent for treating obliteration of blood vessels.

However, urokinase precursor has a problem that it must be used at a relatively high dose because of its short half-life in the blood.

#### SUMMARY OF THE INVENTION

In view of the above, the present inventors conducted many studies to find that its half-life in the blood can be prolonged and its bioavailability can be enhanced by converting urokinase precursor to a lipid composite. Based on the finding, this invention was accomplished.

Thus, this invention provides an urokinase precursor-lipid composite comprising a urokinase precursor and a lipid.

#### BRIEF DESCRIPTION OF THE DRAWING

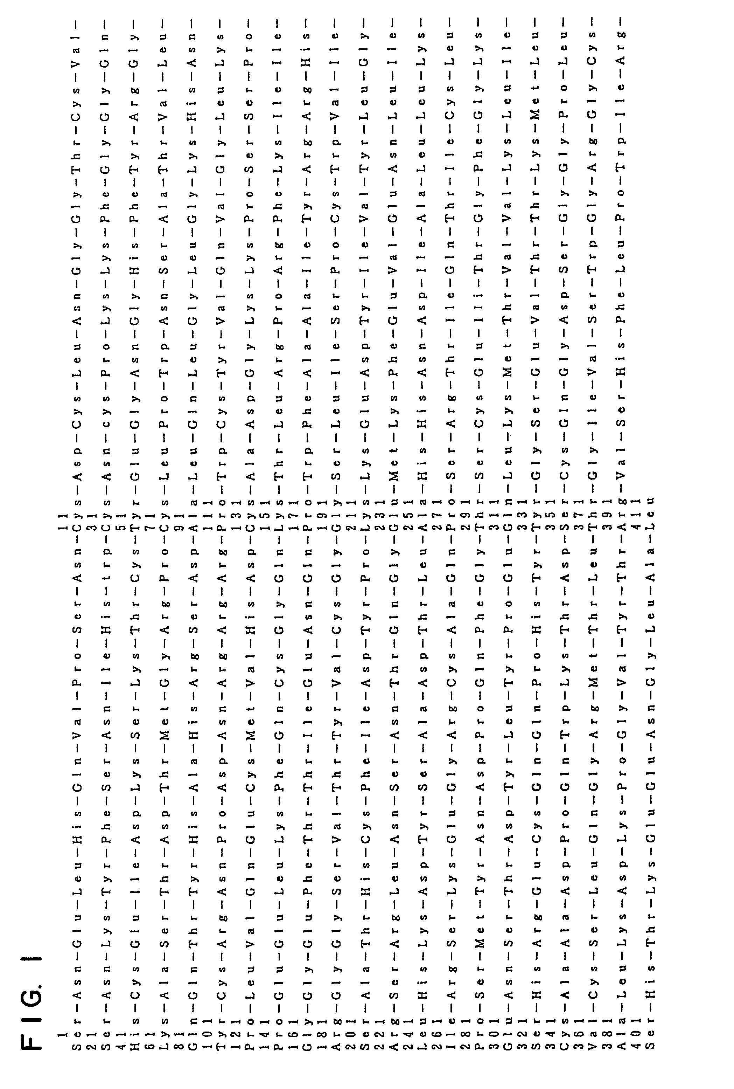

Figure 1 illustrates the amino acid sequence in urokinase precursor, though it will be apparent to those skilled in the art that various modifications (such as amino acid deletions, insertions, additions, substitutions, replacements and inversions) to the nature of the protein can be made (for example as hereinafter described) and that natural allelic variants may arise, which modifications and variants retain essentially the same biological activity. A composite comprising a lipid and any such urokinase precursor falls within the invention.

#### DESCRIPTION OF THE PREFERRED EMBODIMENTS

(i) Urokinase precusor

The urokinase precursor used in this invention scarcely has fibrinolytic activity per se. However, if treated with plasmin or the like, it is converted to (double chain) urokinase and exhibits a fibrinolytic activity. Further, it exhibits a certain extent of fibrinolytic activity in the presence of fibrin, too.

The first typical example of the single chain urokinase used in this invention has a molecular weight of 50,000 to 55,000 and a single chain peptide bond structure.

As example of such urokinase precursor, a urokinase having 411 constitutional amino acids (regarding its amino acid sequence, see the paragraph of "preparation of urokinase precursor" in the Referential Example mentioned later) can be referred to [cf. Japanese Patent Application Kokai (Laid-Open) No. 60-

2

62981 (EP-B-O 535847)].

Origin of the urokinase precursor is not critical. For example, urokinases produced according to cell culture process, genetic engineering process and the like can be used in this invention. The cell culture process is disclosed in Japanese Patent Application Kokai (Laid-Open) No. 60-62981 (EP-B-O 535847) and the genetic engineering process is disclosed in Japanese Patent Application Kokai (Laid-Open) No. 60-180591 (EP-A-O 154272).

The urokinase precursor referred to in this invention is not limited to those mentioned above, but includes its derivatives. Said derivatives of urokinase precursor include protein molecules in which the epidermal growth factor domain of urokinase precursor is wholly or partially lost, protein molecules in which the epidermal growth factor domain of urokinase precursor is wholly or partially substituted by other amino acid residues, and the like [Japanese Patent Application Kokai (Laid-Open) No. 63-146789 (EP-A-O 253241)]. As used in this invention, therefore, the term "urokinase precursor" inclusively means urokinase precursor itself and the above-mentioned derivatives of urokinase precursor.

Usually, this urokinase precursor derivative has a molecular weight of about 40,000 to 50,000 and has a single chain peptide bond structure similarly to urokinase precursor itself. Further, the mode of exhibition of its fibrinolytic activity is similar to that of urokinase precursor itself.

This urokinase precursor derivative is produced by, for example, the genetic engineering process.

If specific activity of urokinase precursor is measured by the synthesic substrate method, the result does not directly express the activity. Thus, an activity of about 100 to 1,000 UK unit/mg is given in the presence of fibrin, and an activity of about 80,000 to 200,000 UK unit/mg is given after treatment with plasmin.

### (ii) Lipid

The lipid used in this invention is not critical, so far as it can form a liposome. As such lipid, phospholipid, glycolipid, derived lipid, fatty acid and the like can be referred to.

The phospholipid used for forming the composite of this invention may be any phospholipid, so far as it is a non-toxic phospholipid which is physiologically acceptable and can be metabolized. Examples of said phospholipid include phosphatidylcholine, phosphatidylserine, phosphatidic acid, phosphatidylglycerine, phosphatidylethanolamine, phosphatidylinositol, sphingomyelin, dicetyl phosphate, lyso-phosphatidylcholine (lysolecithin) and the like. Soybean phospholipid, yolk phospholipid and the like which are mixtures of the above-mentioned phospholipids are also usable. Among these phospholipids, soybean phospholipid and yolk phospholipid are preferable.

As examples of said glycolipid, cerebroside, sulfatide, ganglioside and the like can be referred to.

As examples of said derived lipid, cholic acid, deoxycholic acid and the like can be referred to.

As examples of said fatty acid, oleic acid, lauric acid, myristic acid, palmitic acid, stearic acid and the like can be referred to.

As the structure of said liposome, multilamellar vesicle (MLV), small unilamellar vesicle, large unilamellar vesicle, reverse phase evaporation vesicle and the like can be referred to.

### (iii) Composite formation

Next, formation of composite will be concretely explained with reference to the case of phospholipid.

Phospholipid is dissolved into a solvent such as chloroform, ethanol or the like and thoroughly homogenized. Then, the solvent is distilled off by drying the vessel under reduced pressure to deposit a thin film of the phospholipid onto inner surface of the vessel. Preferably, prior to the deposition of phospholipid, an antioxidant such as tocopherol (vitamin E) is added to the phospholipid in an amount of about 0.01 to 0.5% (w/w) based on the weight of phospholipid for the purpose of stabilizing the phospholipid.

The urokinase precursor of (i) is dissolved into a buffer solution (for example, citric acid buffer, phosphate buffer, acetate buffer, biological saline water, and the like) of which pH value had been adjusted to 4-11 (preferably 6-7), and the resulting solution is contacted with the above-mentioned thin film of phospholipid and rapidly shaken or stirred. When the lipid constituting the liposome film contains a negatively charged lipid, the urokinase precursor may be added before formation of liposome. If desired, a heat-treatment may be carried out after addition of the urokinase precursor at 40-90°C for several minutes to one hour, for example. The solution of urokinase precursor is used in an amount of 0.0001 to 1 part by weight, as expressed in terms of the weight of protein, per one part of the phospholipid used for formation of thin film.

In preparing the thin film of phospholipid, a stabilizer such as cholesterol, phosphatidic acid, dicetyl phosphate, stearylamine, fatty acid (e.g. palmitic acid) and the like may be added.

Further, the composition of this invention may contain as a stabilizer, a polymeric substance selected from the group consisting of albumin, dextran, vinyl polymer, nonionic surfactant, gelatin and hydroxyethyl starch.

Said polymeric stabilizer may be either incorporated into the gaps in liposome together with active ingredient or added to the liposome composition into which active ingredient has already been incorporated (in other words, added to outside of the liposome). Needless to say, it is also allowable to add the polymeric stabilizer to both inside and outside of the liposome.

Said stabilizer is added in an amount of 0.5 to 10 parts by weight and preferably 1 to 5 parts by weight per one part by weight of lipid.

Preferably, the product is subjected to an ultrasonic treatment (sonication) to adjust its particle diameter to 3 microns or less in the next step. The ultrasonic treatment is carried out at a temperature of 0°C to 70°C, preferably 35°C to 45°C, for a period of about 1 to 60 minutes.

In the above-mentioned manner, there is formed a urokinase precursor-phospholipid composite.

In the composite, proportion of urokinase precursor to lipid is 0.05 to 50 parts by weight, preferably 0.5 to 5 parts by weight, of urokinase precursor per one part by weight of lipid.

A particle diameter of the composite is about 0.02 to 3 microns, and preferably about 0.025 to 0.1 micron.

The composite can be isolated and purified by the well known means such as centrifugation, gel filtration, and the like.

In the next step, the composite is washed with physiologically acceptable aqueous solution if desired, and then it is sterilized, filtered and dividingly poured into vials to obtain a liquid composition, a pellet-form composition or a suspension-form composition. The procedure for making these compositions may be carried out according to the methods widely known in the field of medical drugs. A freeze-dried composition prepared by freezing a liquid composition and then drying it under reduced pressure can also be provided.

The composite of this invention is used as a thrombolytic agent for treating thrombic and obstructive diseases such as cerebral thrombosis, periphero-arterial obstruction, periphero-venous obstruction, acute myocardial infarction, etc.

Its dose is about $10^2$ to $10^7$ UK units, as expressed in terms of "quantity after transformation to urokinase", per one administration per one adult patient, and may be varied depending on the symptoms. As the route of administration, intravenous injection, intravenous instillation, etc. can be referred to.

As compared with urokinase itself, the urokinase precursor-lipid composite of this invention is more prevented from disappearance in the living body, particularly in the blood, and its in-blood concentration can be more enhanced, and its half lifetime in blood becomes controllable.

In addition to the original properties of urokinase precursor itself, therefore, the urokinase precursor-lipid composite of this invention can exhibit a more improved fibrinolytic activity than that of urokinase precursor itself without causing abnormal acceleration of fibrinolytic activity (general fibrinolysis).

Next, this invention will be explained more concretely by way of the following examples and experimental examples. This invention is by no means limited by these examples.

Abbreviations used in these examples have the following significances:

BPS: bovine brain phosphatidylserine
EPC: egg phosphatidylcholine
GTBA: gelatin Tris(HCl) buffered saline A
GTBB: gelatin Tris(HCl) buffered saline B
MLV: multilamellar vesicle
PL: phospholipid
PPA: plasminogen proactivator
SUV: small unilamellar vesicle
UK: urokinase
PPA: urokinase precursor
titer: 2,111 U/ml
specific activity: 420 U/mg-protein
(150,000 UK units corresponds to 450 U; both titer and specific activity mean the values after transformation of urokinase precursor into urokinase).

4

Example 1

Preparation of liposome (MLV)

Into a round-bottomed flask having a capacity of 25 ml was charged 300 micromoles of EPC dissolved in chloroform. By evaporating off the solvent by the use of rotary evaporator, a thin film of lipid was prepared. It was once dissolved into chloroform and again formed into a thin film by evaporating off the chloroform by the use of rotary evaporator. This procedure of dissolution-evaporation was three times repeated. Then, the flask was placed in a vacuum desiccator and evacuated by means of vacuum pump for a period of one hour. Then, 0.6 ml of PPA (2,111 U/ml) was introduced into the flask and made into a suspension by means of Vortex mixer.

Sonication

After setting the round-bottomed flask containing lipid suspension on a sonicator, the liquid phase of test tube was dipped in the water of thermostatted water bath and sonicated. The sonication was carried out with a probe type sonicator at a liquid temperature of 50°C or below.

Gel filtration

A liposome dispersion was subjected to gel filtration in the following manner. Thus, gel filtration was carried out by the use of a column ($\phi$1.0 x 18 cm) packed with Sephacryl S-400 Gel which had been equilibrated against gelatin-containing Tris buffer (pH 7.4). Lipid-containing fractions having been eluted just after the elution of void volume were recovered and named "Liposome 1".

Example 2

The procedure of Example 1 was repeated, except that the 300 micromoles of EPC used in Example 1 was replaced with 240 micromoles of EPC plus 60 micromoles of BPS. A dry product was obtained similarly, and it was named "Liposome 2".

Example 3

A thin film of lipid was prepared in the same manner as in Example 1, using 300 micromoles of EPC and 150 micromoles of oleic acid. After suspending it into 1 ml of buffer solution, it was subjected to sonication to obtain a liposome having a particle diameter of about 50 nm. It was mixed with 0.75 ml of PPA (2,000 U/ml) and allowed to stand at 4°C for one day. It was named "Liposome 3".

Experimental Example 1 (characteristic properties)

Using the eluted lipid fractions obtained in Examples 1 and 2, characteristic properties of composite (liposome) were investigated.

Measurement of phospholipid concentration

Equal quantities of liposome and 10% Triton X-100 solution were mixed together and heated at 60°C for 10 minutes. Using the mixture thus obtained as test sample, concentration of phospholipid was measured by the use of commercial phospholipid measurement kit. As standard sample, choline chloride solution was used. Thus, it was mixed with Triton X-100 solution so as to have the same Triton X-100 concentration as in test sample, from which a calibration curve was prepared.

Measurement of PPA activity

(1) Gelatin buffer solution, pH 8.4 (GTBA)

Into 700 ml of distilled water were dissolved 12.1 g of trishydroxymethylaminomethane, 5.8 g of sodium chloride and 0.5 g of sodium azide. On the other hand, 10.0 g of acid-treated gelatin was dissolved into 200 ml of distilled water with heating. Both the solutions were mixed, temperature of the mixture was adjusted to 25°C, its pH was adjusted to 8.4 by adding dilute hydrochloric acid, and then its volume was adjusted to 1,000 ml by adding distilled water. The diluted solution was sterilized with high-pressure steam at 121°C for 20 minutes and preserved in a tightly stoppered container.

(2) Peptide MCA solution

Five milligrams of glutaryl-glycyl-arginine methylcoumarylamide was dissolved into 1 ml of dimethyl sulfoxide, and diluted to a total volume of 40 ml by adding gelatin buffer solution (pH 8.4). This solution was prepared just before use (0.25 mM).

(3) Plasmin solution

According to nominal unit number, a plasmin containing no urokinase was dissolved into gelatin buffer solution (pH 8.4). When a plasma-containing sample is subjected to measurement, concentration of plasmin varies with the dilution rate of plasma. In this experiment, a plasmin concentration of 2.4 CU/ml, 0.8 CU/ml and 0.2 CU/ml was adopted at a dilution rate of x3, x9 and x45, respectively. This solution was prepared just before use.

(4) Triton solution

Ten grams of Triton X-100 was dissolved into 80 ml of distilled water and then total volume was adjusted to 100 ml by adding distilled water.

(5) Standard solution

Just before use, original solution of PPA (2,111 U/ml) was appropriately diluted with a diluted (x3 or x9) pooled plasma to prepare 0, 0.2, 0.4, 0.6, 0.8, 1.0, 1.2, 1.4, 1.6, 1.8 and 2.0 U/ml solutions. The degrees of dilution with plasma adopted in the serial dilution of standard solution were identical with those adopted in the serial dilution of sample solution.

(6) Sample solution

Test sample was diluted with GTBA 3 times, 9 times or 45 times.

(7) Surfactant treatment

Ninety microliters of standard solution or sample solution was mixed with 10 microliters of 10% Triton X-100 and heat-treated at 60°C for 10 minutes. This was carried out just before use.

(8) Procedure

Into each well of a microplate having 96 wells, 25 microliters of sample solution or standard solution which had been treated or not treated with surfactant and 25 microliters of 0.2 CU/ml plasmin solution were

6

introduced and kept at 37°C for 10 minutes. After cooling the microplate in ice water, 100 microliters of 0.25 mM GGA-MCA solution was added, and then the microplate was kept at 37°C for 20 minutes. After cooling the microplate with ice water, 50 microliters of 60% acetic acid solution was added, and the fluorescent value was read out by means of microplate reader (wavelength of excitation: 365 nm; wavelength of fluorescence: 450 nm).

Table 1 summarizes phospholipid concentration of liposome, PPA activity of surfactant-treated system, and its yield.

Table 1.

| Phospholipid concentration, PPA activity and yield | | | | |
|---|---|---|---|---|
| Liposome | Phospholipid | | PPA activity | |
| | μmole/ml | % | U/ml | % |
| No. 1 | 40.6 | 60.9 | 46.5 | 16.5 |
| No. 2 | 43.0 | 64.5 | 69.6 | 24.7 |
| No. 3 | 71.3 | 95.0 | 352.7 | 94.1 |

Table 2 illustrates ratio (percentage) of PPA activity in the surfactant-untreated system to PPA activity in the surfactant-treated system.

Table 2.

| Proportion (%) of PPA activity which can be exhibited without surfactant treatment | |
|---|---|
| Liposome | Proportion (%) |
| No. 1 | 8.7 |
| No. 2 | 97.3 |
| No. 3 | 99.8 |

Measurement of liposome particle diameter

After diluting 20 to 50 microliters of liposome sample with 2 ml of 0.15M sodium chloride solution, average particle diameter of liposome was measured. As the result, it was 70.5 nm in Liposome 1, 89 nm in Liposome 2, and 51.6 nm in Liposome 3.

Experimental Example 2

Plasma clearance following intravenous administration was investigated in rat.

Male Wistar rats (body weight 130 g, 3 heads per one group) were used. Concentration of PPA in the liposome to be administered to rats was adjusted to 40 U/ml. Concentration of PPA used as control was 400 U/ml. Three heads of rats were used as one group, and one milliliter of sample solution was injected into the vein of tail. Before administration and 2, 15, 30, 60 and 300 (only in case of liposome administration) minutes after administration, 0.5 ml of blood was sampled out. The sampled blood was mixed with its 1/10 quantity of 3% sodium citrate solution and immediately centrifuged at 2,000 rpm for 20 minutes to separate the plasma. The plasma was diluted with GTBA 3 times, 9 times and 45 times, and the dilutions were stored at room temperature till the measurement of PPA activity. Measurement of PPA activity in surfactant-treated and surfactant-untreated systems was carried out in the same manner as in Experimental Example 1, provided that the measurement of PPA activity in the untreated system was carried out within one hour after sampling the blood.

The results are shown in tables 3, 4 and 5.

Table 3

| Time after administration (minutes) | PPA activity (U/ml) | | |
|---|---|---|---|
| | Control | Intravenous injection of PPA-containing lipsome No. 1 | |
| | Intravenous injection of PPA itself | Untreated with surfactant | Treated with surfactant |
| 2 | 104.6 | 0.531 | 6.75 |
| 15 | 3.95 | 0.240 | 7.01 |
| 30 | 0.95 | 0.220 | 6.28 |
| 60 | 0.12 | 0.227 | 4.79 |
| 300 | < 0.01 | 0.122 | 3.22 |

Table 4

| Time after administration (minutes) | PPA activity (U/ml) | | |
|---|---|---|---|
| | Control | Intravenous injection of PPA-containing liposome No. 2 | |
| | Intravenous injection of PPA itself | Untreated with surfactant | Treated with surfactant |
| 2 | 104.6 | 4.08 | 5.80 |
| 15 | 3.95 | 3.13 | 4.71 |
| 30 | 0.95 | 2.35 | 3.01 |
| 60 | 0.12 | 1.60 | 2.40 |
| 300 | < 0.01 | 0.41 | 0.89 |

Table 5

| Time after administration (minutes) | PPA activity (U/ml) | | |
|---|---|---|---|
| | Control | Intravenous injection of PPA-containing liposome No. 3 | |
| | Intravenous injection of PPA itself | Untreated with surfactant | Treated with surfactant |
| 2 | 104.6 | 15.8 | 15.9 |
| 15 | 3.95 | 4.32 | 4.33 |
| 30 | 0.95 | 2.95 | 2.95 |
| 60 | 0.12 | 1.95 | 1.95 |
| 300 | < 0.01 | 0.23 | 0.23 |

Experimental Example 3

Thrombolytic activity in pulmonary embolism model was investigated in rat.

Fibrin clot labelled with [125]I was administerd to anesthetized rats from the tail vein to cause pulmonary embolism. Five minutes after, Liposome No. 3 or PPA (control) was administered in the form of bolus (5 heads per one group). One hour after the administration of fibrin clot, the animals were slaughtered and radio-activity of [125]I-fibrin remaining in the lungs was measured, from which rate of thrombolysis was determined. The results are summarized in Table 6.

Table 6

| Agent | Dose (IU/kg) | Rate of thrombolysis | |
|---|---|---|---|
| | | Mean value ± S.E. | |
| Vehicle | 0 | 45.0 | 5.58 |
| PPA | 100,000 | 54.5 | 2.66 |
| PPA | 200,000 | 64.2* | 2.49 |
| PPA | 300,000 | 75.6** | 1.50 |
| Liposome No. 3 | 100,000 | 77.9**## | 2.21 |

* $P < 0.05$

** $P < 0.01$ vs. Vehicle group

## $P < 0.01$ vs. PPA 100,000 IU/kg group

Referential Example

Preparation of urokinase precursor

Cultured cells of human kidney were cultured for 3 days in a serum-free culture medium containing 0.1% human serum albumin. The cultured mixture was centrifuged, and the supernatant was freezed and stored. After adjusting the pooled supernatant to pH 5.5, it was contacted with CM-Sephadex C-50. After washing the column with 0.16M phosphate buffer (pH 5.5), the adsorbed urokinase precursor was eluted with 0.16M phosphate buffer (pH 8.5).

On the other hand, spleen cells of mouse BALB/c and mouse myeloma cells, having previously been immunized with urokinase precursor, were fused together by the use of polyethylene glycol to prepare a hybridoma, from which a clone showing a high antibody production against urokinase precursor was screened out. From a culture fluid of this fused cell, urokinase precursor monoclonal antibody was recovered. This monoclonal antibody was immobilized on BrCN-activated Sepharose 4B (manufactured by Pharmacia Co.).

This monoclonal antibody column was equilibrated against 0.1M phosphate buffer (pH 7.0) containing 0.4M sodium chloride, and it was contacted with the above-mentioned eluted solution containing urokinase precursor. After washing the column with 0.1M phosphate buffer (pH 7.0) containing 0.4M sodium chloride, the adsorbed urokinase precursor was eluted with 0.2M aqueous solution of glycine-hydrochloride containing 0.5M sodium chloride (pH 2.5). After sterilizing and filtering the eluted solution, it was freeze-dried to obtain a highly purified urokinase precursor having a specific activity of 150,000 UK unit/mg-protein (in terms of activity affer transformation to urokinase).

This purified product showed a single band of molecular weight 54,000 in SDS-polyacrylamide gel electrophoresis. Its amino acid sequence was as shown in Figure 1.

## Claims

1. A urokinase precursor-lipid composite comprising urokinase precursor and a lipid.

2. A composite according to Claim 1, wherein said urokinase precursor has a molecular weight of 50,000 to 55,000 and a single chain peptide bond structure.

3. A composite according to Claim 1 or 2, wherein said urokinase precursor has an amino acid sequence shown in Figure 1.

4. A composite according to any preceding claim, wherein said urokinase precursor has a specific activity of 100 to 1,000 UK units/mg in the presence of fibrin and 80,000 to 200,000 UK units/mg when treated with plasmin.

5. A composite according to any preceding claim, wherein said lipid is a phospholipid, a glycolipid or a derived lipid which can form a liposome.

6. A composite according to Claim 5, wherein said phospholipid is a soybean phospholipid or an egg yolk phospholipid.

7. A composite according to Claim 5, wherein said glycolipid is cerebroside, sulfatide or ganglioside.

8. A composite according to Claim 5, wherein said derived lipid is cholic acid or deoxycholic acid.

9. A composite according to any preceding claim, wherein said composite has a particle diameter of 0.02 to 3 microns.

10. A pharmaceutical composition comprising a composite according to any preceding claim and a pharmaceutically acceptable excipient.

# FIG. I

Ser-Asn-Glu-Leu-His-Gln-Val-Pro-Ser-Asn-Cys-Asp-Cys-Leu-Asn-Gly-Gly-Thr-Cys-Val-
Ser-Asn-Lys-Tyr-Phe-Ser-Asn-Ile-His-trp-Cys-Asn-cys-Pro-Lys-Lys-Phe-Gly-Gly-Gln-
His-Cys-Glu-Ile-Asp-Lys-Ser-Lys-Thr-Cys-Tyr-Glu-Gly-Asn-Gly-His-Phe-Tyr-Arg-Gly-
Lys-Ala-Ser-Thr-Asp-Thr-Met-Gly-Arg-Pro-Cys-Leu-Pro-Trp-Asn-Ser-Ala-Thr-Val-Leu-
Gln-Gln-Thr-Tyr-His-Ala-His-Arg-Ser-Asp-Ala-Leu-Gln-Leu-Gly-Leu-Gly-Lys-His-Asn-
Tyr-Cys-Arg-Asn-Pro-Asp-Asn-Arg-Arg-Arg-Pro-Trp-Cys-Tyr-Val-Gln-Val-Gly-Leu-Lys-
Pro-Leu-Val-Gln-Glu-Cys-Met-Val-His-Asp-Cys-Ala-Asp-Gly-Lys-Lys-Pro-Ser-Ser-Pro-
Pro-Glu-Glu-Leu-Lys-Phe-Gln-Cys-Gly-Gln-Lys-Thr-Leu-Arg-Pro-Arg-Phe-Lys-Ile-Ile-
Gly-Gly-Glu-Phe-Thr-Thr-Ile-Glu-Asn-Gln-Pro-Trp-Phe-Ala-Ala-Ile-Tyr-Arg-Arg-His-
Arg-Gly-Gly-Ser-Val-Thr-Tyr-Val-Cys-Gly-Gly-Ser-Leu-Ile-Ser-Pro-Cys-Trp-Val-Ile-
Ser-Ala-Thr-His-Cys-Phe-Ile-Asp-Tyr-Pro-Lys-Lys-Glu-Asp-Tyr-Ile-Val-Tyr-Leu-Gly-
Arg-Ser-Arg-Leu-Asn-Ser-Asn-Thr-Gln-Gly-Glu-Met-Lys-Phe-Glu-Val-Glu-Asn-Leu-Ile-
Leu-His-Lys-Asp-Tyr-Ser-Ala-Asp-Thr-Leu-Ala-His-His-Asn-Asp-Ile-Ala-Leu-Leu-Lys-
Ile-Arg-Ser-Lys-Glu-Gly-Arg-Cys-Ala-Gln-Pro-Ser-Arg-Thr-Ile-Gln-Thr-Ile-Cys-Leu-
Pro-Ser-Met-Tyr-Asn-Asp-Pro-Gln-Phe-Gly-Thr-Ser-Cys-Glu-Ili-Thr-Gly-Phe-Gly-Lys-
Glu-Asn-Ser-Thr-Asp-Tyr-Leu-Tyr-Pro-Glu-Gln-Leu-Lys-Met-Thr-Val-Val-Lys-Leu-Ile-
Ser-His-Arg-Glu-Cys-Gln-Gln-Pro-His-Tyr-Tyr-Gly-Ser-Glu-Val-Thr-Thr-Lys-Met-Leu-
Cys-Ala-Ala-Asp-Pro-Gln-Trp-Lys-Thr-Asp-Ser-Cys-Gln-Gly-Asp-Ser-Gly-Gly-Pro-Leu-
Val-Cys-Ser-Leu-Gln-Gly-Arg-Met-Thr-Leu-Thr-Gly-Ile-Val-Ser-Trp-Gly-Arg-Gly-Cys-
Ala-Leu-Lys-Asp-Lys-Pro-Gly-Val-Tyr-Thr-Arg-Val-Ser-His-Phe-Leu-Pro-Trp-Ile-Arg-
Ser-His-Thr-Lys-Glu-Glu-Asn-Gly-Leu-Ala-Leu

EP 0 380 334 A2